# EUROPEAN PATENT SPECIFICATION

(11) **EP 0 689 832 B1**
(45) Date of publication and mention of the grant of the patent: **27.09.2000**
(21) Application number: 95500092.2
(22) Date of filing: 28.06.1995
(51) Int. Cl.: A61K 9/08, A61K 31/495

(54) **Antibiotic preparation for aural application**
Antibiotische Zubereitung zur auralen Anwendung
Préparation antibiotique pour application auriculaire

(30) Priority: 29.06.1994 ES 9401411
(43) Date of publication of application: 03.01.1996
(73) Proprietor: LABORATORIOS S.A.L.V.A.T., S.A., 08950 Esplugues de Llobregat (ES)
(72) Inventor: Montserrat Vidal, Carlos, E-08950 Esplugues de Llobregat (ES)
(74) Representative: SUGRANES - VERDONCES - FERREGÜELA

(56) References cited:
- WO-A-90/01933
- DE-A- 3 632 222
- US-A- 4 957 922
- CHOWDARY K.P.R. ET AL: "Release and antimicrobial activity of ciprofloxacin from topical drug delivery systems" EAST. PHARM., vol. 38, no. 449, May 1995, pages 145-146, XP002051940
- SUCKER H. ET AL: "Pharmazeutische Technologie" 1991 , THIEME VERLAG , STUTTGART XP002051941 189560 * page 670 - page 672 *

## Description

The present invention refers to a preparation of the antibiotic ciprofloxacin suitable for the effective treatment of otitis, this preparation being highly effective and having a low risk of ototoxicity. One substantial difference with respect to other known aural preparations of antibiotics is that it can be used with children.

The therapeutic treatment of otitis (inflammation of the ear) is a problem which has not been satisfactorily resolved, especially in children. Otitis media, or inflammation of the middle ear, which is very frequent in childhood, is generally accompanied by otorrhoea (mucopurulent secretion). Otitis externa is inflammation of the auditory canal which generally reveals itself as dermoepidermitis. In both types of otitis the germs responsible are frequently of the gram-negative bacillus type, particularly Pseudomonas aeruginosa.

It is well known that most patients lend themselves more readily to localized topical administration of a drug than to oral or parenteral administration. Furthermore, certain antibiotics cannot be administered orally or parenterally to children; thus, for example, many of the modern antibiotics are not recommended for children under 14 years.

Some topical preparations of antibiotics have been used in the treatment of otitis. However, hardly any antibiotics other than those of the aminoglycosides group (neomycin, framycetin, gentamicin) or the tetracyclines group have been used in this form of administration. It is nevertheless known that when these antibiotics are administered topically in cases of perforation of the ear drum there is a high risk of ototoxicity (cf., for example, P. Morrell et al., "Deafness in preterm baby associated with topical antibiotic spray containing neomycin", Lancet 1985, 1 (8438), p. 1167-8; T. Harada et al., "Ototoxicity of neomycin and its penetration through the round window membrane into the perilymph" Ann. Otol. Rhinol. Laryngol. 1986, vol. 95, p. 404-8).

So no satisfactory solution has yet been found for the problem of administration in the form of aural drops of preparations of antibiotics sufficiently active to combat the various forms of otitis effectively, and sufficiently safe to minimize the risk of ototoxicity in cases of perforation of the ear drum. The present invention resolves this problem by providing antibiotic preparations for aural administration, for which, amongst the numerous known antibiotics, that known as ciprofloxacin (INN) or 1-cyclopropyl-6-fluoro-1,4-dihydride-4-oxo-7-(1-piperazinyl)-3-quinolyncarboxylic has been chosen, it being an antibiotic described chemically for the first time in 1983 (DE 3.142.854 A).

Ciprofloxacin is a broad-spectrum antibiotic belonging to the new generation of quinolones, which has shown itself to be very effective in the treatment of systemic infections, and rarely develops bacterial resistance. Oral and parenteral administration are the usual routes for administering ciprofloxacin, in the form of hydrochloride or lactate, although its use in ophthalmic preparations has been mentioned (US 4.865.846 and WO 9001933). Toxicological studies on animals show that the product has no significant toxicity, although as a precaution it is recommended to refrain from using it for the time being with children under four years of age.

Preparations of ciprofloxacin suitable for topical administration in the ear are known neither in the literature nor in the market. US patent 4.957.922 describes solutions of ciprofloxacin for the treatment of systemic or local infections, while in column 6 it cites otitis among over fifty other indications which include microbial infections of practically the whole human body. In fact, however, of the nearly one hundred preparations described in the examples of US patent 4.957.922, none is designed for aural application. Of them all, the preparation which is closest to that of the present invention is no. 2, in which for every 100 ml of aqueous solution there are 25 mg of ciprofloxacin, 93 mg of lactic acid of 10% by weight, 1.3 mg of hydrochloric acid and 5g of glucose. This solution is much more dilute than is advisable for aural application, and lacks some ingredients essential for this application.

The object of the present invention is thus to provide an antibiotic preparation for aural application which, together with a quantity of water sufficient to make up to 100 ml of solution, and together with other possible accessory components, includes the following ingredients : between 0.2 and 0.4 g of ciprofloxacin; a quantity of lactic acid equivalent to between 0.3 and 0.8 g of aqueous solution of lactic acid at 20%; a quantity of hydrogen chloride equivalent to between 0.3 and 0.6 g of 1M hydrochloric acid, so that the sum of the above quantities of lactic and hydrochloric acid represents a total quantity of between 1.3 and 2.3 moles of acid per mole of ciprofloxacin; a quantity of between 1 and 4 g of a sugar selected from glucose, fructose, lactose and saccharose; a quantity between 0.04 and 0.08 g of methyl p-hydroxybenzoate; a quantity between 0.02 and 0.04 g of ethyl p-hydroxybenzoate; a quantity between 0.7 and 1.6 g of a co-solvent, either propylene glycol or polyethylene glycol; a quantity between 1 and 4 g of a viscosity agent selected from amongst polyvinyl pyrrolidone, hydroxypropyl cellulose, hydroxypropylmethyl cellulose, carboxypolymethylene and a crosslinked polymer derived from acrylic acid (e.g. that commercialized under the brand name Carbopol ^{(R)}).

In a preferred embodiment of the invention, the approximate quantities of the ingredients are as follows: 0.3 g of ciprofloxacin, 0.55 g of 20% lactic acid, 0.43 g of 1M hydrochloric acid, 2.5 g of sugar, 0.06 g of methyl p-hydroxybenzoate, 0.3 g of ethyl p-hydroxybenzoate, 1.2 g of solvent and 2.5 g of viscosity agent.

Special preference is accorded to the antibiotic preparation for aural application in which the sugar is glucose, the co-solvent is propylene glycol and the viscosity agent is polyvinyl pyrrolidone.

The preparation of the invention is an aqueous solution which is sufficiently fluid for application to the ear using the usual dropper devices.

The simultaneous presence of all the ingredients of the preparation object of the invention achieves a synergic effect, advantageous in that: a) the active ingredient ciprofloxacin is dissolved in a stable and lasting manner; b) the pH of the medium is suitable for aural application thereof; c) the preparation is not chemically aggressive to the ear; d) the osmosity is suitable; e) the solution keeps well in an oxidizing atmosphere and against attack by ambient microorganisms (e.g. fungi), both in its container and once applied to the ear; f) the viscosity is suitable to prevent the solution separating mechanically from the ear; g) the cerumen is softened, which encourages penetration of the preparation in the ear.

One very important advantage of the preparation object of the invention with respect to known preparations for oral or parenteral administration of ciprofloxacin is that it can be applied to children, who frequently suffer from otitis and are problematical to treat.

Use of the aural preparation of this invention, with respect to known aural preparations containing other antibiotics has advantages deriving from the greater antibiotic activity of ciprofloxacin and its lower bacterial resistance.

Due to the absence of ototoxicity of ciprofloxacin, the therapeutic treatment of the various types of otitis by means of the preparation of the invention presents a much lower risk than known preparations of neomycin, gentamicin or tetracycline, in patients with perforation of the ear drum.

The enclosed examples illustrate a preparation in accordance with the present invention, together with its applicability in the treatment of two very common types of otitis. The clinical trial conducted shows that the preparation of this invention has an antibiotic efficacy as good as or better than that of gentamicin, without presenting the toxicity problems of the latter.

### EXAMPLES

### Example 1: ciprofloxacin preparation for aural application

Following the procedures usual in galenical practice, 100 ml of a solution were prepared for topical administration, with the following preparation:

| | |
|---|---|
| ciprofloxacin | 300 mg |
| 20% lactic acid | 555 mg |
| polyvinyl pyrrolidone | 2500 mg |
| anhydrous glucose | 2500 mg |
| propylene glycol | 1160 mg |
| methyl p-hydroxybenzoate | 60 mg |
| propyl p-hydroxybenzoate | 30 mg |
| 1M hydrochloric acid | 432 g |
| distilled water (sufficient quantity for) | 100 ml |

### Example 2: comparative study of ciprofloxacin and gentamicin

Over a 9-month period, in a hospital, with volunteer patients aged 18 years or more, a randomized, double-blind, prospecting study was carried out on the efficacy and tolerance of topical ciprofloxacin compared with topical gentamicin in the treatment of simple chronic suppurative otitis media (53 patients) and diffuse otitis externa (53 patients). Half of the patients were given a preparation like that of Example 1 by topical administration, 5 drops 3 times a day. The other group was given gentamicin topically, in the form of a preparation similar to that of Example 1, but with a concentration of 3 mg/ml, 5 drops 3 times a day.

To assess the results, it was considered on the basis of data from the literature that the efficacy of gentamicin was 68%, while that of ciprofloxacin was 90%. The conclusions of the study were as follows:
a) Despite the fact that no statistically significant differences were found between the treatment with ciprofloxacin and with gentamicin (probably due to the size of the sample), the efficacy of the topical ciprofloxacin preparation under trial can be considered to be very good, both in the treatment of otitis externa and chronic otitis media.
b) No symptom of ototoxicity was observed in the treatment with topical ciprofloxacin.
c) From the clinical point of view, the efficacy of the ciprofloxacin preparation under trial, which was 95% for chronic otitis media and 87% in the case of otitis externa, can be classified as very good. Likewise, the total absence of any undesirable effect (local tolerance; signs or symptoms of cochleovestibular ototoxicity) confirms the impression of total safety of the preparation under trial.

## Claims

1. An antibiotic preparation for aural application which, together with a quantity of water sufficient to make up to 100 ml of solution, and together with other possible accessory components, includes the following ingredients: - between 0.2 and 0.4 g of ciprofloxacin;
- a quantity of lactic acid equivalent to between 0.3 and 0.8 g of aqueous solution of lactic acid at 20%;
- a quantity of hydrogen chloride equivalent to between 0.3 and 0.6 g of 1M hydrochloric acid, so that the sum of the above quantities of lactic acid and hydrogen chloride represents a total quantity of between 1.3 and 2.3 moles of acid per mole of ciprofloxacin;
- a quantity of between 1 and 4 g of a sugar selected from glucose, fructose, lactose and saccharose;
this preparation being characterized in that it also includes:
- a quantity between 0.04 and 0.08 g of methyl p-hydroxybenzoate;
- a quantity between 0.02 and 0.04 g of ethyl p-hydroxybenzoate;
- a quantity between 0.7 and 1.6 g of a co-solvent, either propylene glycol or polyethylene glycol;
- a quantity between 1 and 4 g of a viscosity agent selected from polyvinyl pyrrolidone, hydroxypropyl cellulose, hydroxypropylmethyl cellulose, carboxypolymethylene and a crosslinked polymer derived from acrylic acid.

2. An antibiotic preparation for aural application, as claimed in Claim 1, in which the approximate quantities of the ingredients are as follows: 0.3 g of ciprofloxacin, 0.55 g of 20% lactic acid, 0.43 g of 1M hydrochloric acid, 2.5 g of sugar, 0.06 g of methyl p-hydroxybenzoate, 0.3 g of ethyl p-hydroxybenzoate, 1.2 g of co-solvent and 2.5 g of viscosity agent.

3. An antibiotic preparation for aural application, as claimed in either of Claims 1 and 2, in which the sugar is glucose, the co-solvent is propylene glycol and the viscosity agent is polyvinyl pyrrolidone.

## Patentansprüche

1. Antibiotische Zubereitung zur auralen Anwendung, welche zusammen mit einer ausreichenden Menge Wasser, um 100 ml Lösung vorzusehen, und zusammen mit weiteren möglichen Zusatzkomponenten die folgenden Bestandteile beinhaltet:
- zwischen 0,2 und 0,4 g Ciprofloxacin;
- eine Menge an Milchsäure, welche zwischen 0,3 und 0,8 g einer wässrigen 20%-igen Lösung von Milchsäure äquivalent ist;
- eine Menge an Wasserstoffchlorid, welche zwischen 0,3 und 0,6 g 1M Chlorwasserstoffsäurte äquivalent ist, so daß die Summe der obigen Mengen an Milchsäure und Wasserstoffchlorid eine Gesamtmenge zwischen 1,3 und 2,3 Molen an Säure pro Mol Ciprofloxacin dartstell;
- eine Menge zwischen 1 und 4g eines Zuckers, gewälhlt aus Glucose, Fructose, Lactose und Saccharose;
wobei diese Zubereitung dadurch gekennzeichnet ist, daß sie ebenso beinhaltet:
- eine Menge zwischen 0,04 und 0,08 g Methyl-p-hydroxybenzoat;
- eine Menge zwischen 0,02 und 0,04 g Ethyl-p-hydroxybenzoat;
- eine Menge zwischen 0,7 und 1,6 g eines Co-Lösungsmittels, entweder Propylenglycol oder Polyethylenglycol;
- eine Menge zwischen 1 und 4 g eines Viskositätsmittels, gewählt aus Polyvinylpyrolidon, Hydroxypropylcellulose, Hydroxypropylmethylcellulose, Carboxypolymethylen uns vernetztes Polymer, abgeleitet aus Acrylsäure.

2. Antibiotische Zubereitung zur auralen Anwendung nach Anspruch 1, wobei die ungefähren Mengen der Bestandteile wie folgt sind: 0,3 g Ciprofloxacin, 0,55 g, 20%-ige Milchsäure, 0,43 g 1M Chlorwasserstoffsäure, 2,5 g Zucker, 0,06 g Methyl-p-hydroxybenzoat, 0,3 g Ethyl-p-hydroxybenzoat, 1,2 g Co-Lösungsmittel und 2,5 g Viskositätsmittel.

3. Antibiotische Zubereitung zur auralen Anwendung nach Anspruch 1 und/oder 2, wobei der Zucker Glucose ist, das Co-Lössungsmittel Propylenglycol ist und das Viskositätsmittel Polyvinylpyrrolidon ist.

## Revendications

1. Préparation antibiotique pour administration auriculaire, qui, en même temps qu'une quantité d'eau suffisante pour constituer 100 ml de solution, et en même temps que d'autres éventuels composants accessoires, comprend les ingrédients suivants :
- de 0,2 à 0,4 g de ciprofloxacine ;
- une quantité d'acide lactique équivalente à une quantité de 0,3 à 0,8 g d'une solution aqueuse d'acide lactique à 20 % ;
- une quantité de chlorure d'hydrogène équivalente à une quantité comprise entre 0,3 et 0,6 g d'acide chlorhydrique 1M, de façon que la somme des quantités ci-dessus d'acide lactique et de chlorure d'hydrogène représente une quantité totale de 1,3 à 2,3 moles d'acide par mole de ciprofloxacine ;
- une quantité de 1 à 4 g d'une sucre choisi parmi le glucose, le fructose, le lactose et le saccharose ;
cette préparation étant caractérisée en ce qu'elle contient aussi :
- une quantité de 0,04 à 0,08 g de p-hydroxybenzoate de méthyle ;
- une quantité de 0,02 à 0,04 g de p-hydroxybenzoate d'éthyle ;
- une quantité de 0,7 à 1,6 g d'un co-solvant, qui est le propylèneglycol ou le polyéthylèneglycol ;
- une quantité de 1 à 4 g d'un agent de viscosité choisi parmi la polyvinyl pyrrolidone, l'hydroxypropylcellulose, l'hydroxypropylméthylcellulose, le carboxypolyméthylène et un polymère réticulé dérivé de l'acide acrylique.

2. Préparation antibiotique pour administration auriculaire selon la revendication 1, dans laquelle les quantités approximatives des ingrédients sont les suivantes : 0,3 g de ciprofloxacine, 0,55 g d'acide lactique à 20 %, 0,43 g d'acide chlorhydrique 1M, 2,5 g de sucre, 0,06 g de p-hydroxybenzoate de méthyle, 0,3 g de p-hydroxybenzoate d'éthyle, 1,2 g d'un co-solvant et 2,5 g d'un agent de viscosité.

3. Préparation antibiotique pour administration auriculaire selon l'une des revendications 1 ou 2, dans laquelle le sucre est le glucose, le co-solvant est le propylèneglycol, et l'agent de viscosité est la polyvinyl pyrrolidone.
